# EUROPEAN PATENT SPECIFICATION

(11) **EP 3 668 408 B1**
(45) Date of publication and mention of the grant of the patent: **15.07.2026**
(21) Application number: 18759572.3
(22) Date of filing: 10.08.2018
(51) Int. Cl.: A61B 8/08, A61B 8/00

(54) **ULTRASOUND SYSTEM WITH EXTRACTION OF IMAGE PLANES FROM VOLUME DATA USING TOUCH INTERACTION WITH AN IMAGE**
ULTRASCHALLSYSTEM MIT EXTRAKTION VON BILDEBENEN AUS VOLUMENDATEN UNTER VERWENDUNG EINER BERÜHRUNGSINTERAKTION MIT EINEM BILD
SYSTÈME À ULTRASONS CAPABLE D'EXTRACTION DE PLANS D'IMAGE À PARTIR DE DONNÉES DE VOLUME PAR INTERACTION PAR EFFLEUREMENT AVEC UNE IMAGE

(30) Priority: 17.08.2017 US 201762546590 P
(43) Date of publication of application: 24.06.2020
(73) Proprietor: Koninklijke Philips N.V., 5656 AE Eindhoven (NL)
(72) Inventor: ROUNDHILL, David Nigel, 5656 AE Eindhoven (NL)
(74) Representative: Philips Intellectual Property & Standards
(86) International application number: PCT/EP2018/071721
(87) International publication number: WO 2019/034546

(56) References cited:
- EP-A1- 2 965 693
- EP-A1- 2 982 306
- EP-A1- 3 115 000
- EP-A2- 2 434 454
- KR-A- 20170 054 982
- US-A1- 2014 114 190
- NIKOONAHAD M ET AL: "MEDICAL ULTRASOUND IMAGING USING NEURAL NETWORKS", ELECTRONICS LETTERS, IEE STEVENAGE, GB, vol. 26, no. 8, 14 April 1990 (1990-04-14), pages 545/546, XP000120906, ISSN: 0013-5194

## Description

This invention relates to medical diagnostic ultrasound systems and, in particular, to ultrasound systems which enable the extraction of image planes of selected anatomy by touch interaction with an image.

Obstetrical fetal imaging is one of the most important branches of ultrasonic imaging. Ultrasound is a non-ionizing imaging modality and hence safe for developing fetuses. Ultrasound imaging is used to monitor fetal development and also to predict expected delivery dates from estimations of fetal age. Fetal age estimation is done by measuring the dimensions of various bones of a developing fetus, such as the skull and limbs. Clinically validated algorithms use these measurements in combination to estimate fetal age. A typical ultrasound system configured for obstetrical imaging is equipped with protocols to guide a sonographer in acquiring the necessary images for the measurements and also will have the age estimation algorithms onboard the system.

Acquiring the necessary images for accurate measurements of the fetal bone structure is not always easy, however. The most accurate measurements are made when the longitudinal dimension of a limb bone is fully captured in a two-dimensional (2D) image plane, but manipulating the image plane with a standard 2D imaging probe is often problematic. The fetus can move frequently and can assume various positions in the womb, putting the limbs into orientations which are not always accessible to the 2D plane extending from the probe. A solution for this situation is provided by 3D volumetric imaging. The 3D volumetric region extending from the aperture of a 3D imaging probe can be positioned to capture the volume in which the fetus is located, regardless of the current position of the fetus. A volume image can be captured quickly and then analyzed and diagnosed at leisure by viewing different slice planes of the volume using multiplanar reformatting to extract desired planes containing the necessary bones for measurement. But a problem often encountered in such extraction is that the desired anatomy may not always be clearly visible, as it can be obstructed by surrounding tissue, the umbilical cord, or the wall of the uterus. Thus it sometimes becomes necessary to "trim" away obscuring tissue in a volume image and search carefully to find the images of the tiny structures required for measurements. It is desirable to expedite this process so that the desired anatomy can be viewed quickly and completely for accurate measurement.

EP 3115000 discloses an ultrasound diagnosis apparatus in which a user input is received selecting a reference object included in a displayed reference cross-section of an object extracted from volume data, and wherein the volume data is normalized by using location information of the reference object.

US 2014/114190 A1 discloses a touch screen display for displaying an ultrasound image, and a method comprising the steps of: receiving a first gesture input from the touch screen display and in response to the first gesture input from the touch screen display, altering a display operation.

The present invention is defined by the independent claims. Advantageous embodiments are provided in the dependent claims.

In accordance with the principles of the present invention, an ultrasound system enables extraction of image planes of desired anatomy from a 3D volume image dataset using image processing which has been programmed to identify the desired anatomy in 3D volume images. A 3D volume image dataset is acquired and an ultrasound image displayed on a touchscreen display. When a user sees a section of desired anatomy in an ultrasound image, the user touches the anatomy on the touchscreen. This sends a cue to an image extraction processor, which then examines image planes around the identified anatomical location and locates an image plane containing the desired anatomy. An image of the identified anatomy is displayed to a user, and the display may also automatically include a measurement of the desired anatomy useful for a particular exam such as a fetal exam.

In the drawings:
FIGURE 1 illustrates a tablet ultrasound system with a touchscreen display and an ultrasound probe for use with the system.
FIGURE 2 illustrates a 3D volume image containing a fetal femur and a cut plane intersecting the femur.
FIGURE 3 is a picture of a femur indicating the location on the bone where it is intersected by the cut plane when imaged with a volume image as shown in FIGURE 2.
FIGURE 4 is an ultrasound image display showing an image of the cut plane of FIGURE 2.
FIGURE 5 is a block diagram of an ultrasound system constructed in accordance with a first implementation, provided for illustration purposes and not within the scope of the claims, which uses a fetal model to identify the image planes of fetal bones in volume image data.
FIGURE 6 is a flowchart illustrating the operation of the ultrasound system of FIGURE 5.
FIGURE 7 is an ultrasound display of a fetal bone in a longitudinal view suitable for measurement which has been identified and extracted in accordance with the principles of the present invention.
FIGURE 8 is a block diagram of an ultrasound system constructed in accordance with a second implementation of the present invention which uses a neural network model to identify the image planes of fetal bones in volume image data.
FIGURE 9 is a flowchart illustrating the operation of the ultrasound system of FIGURE 8.

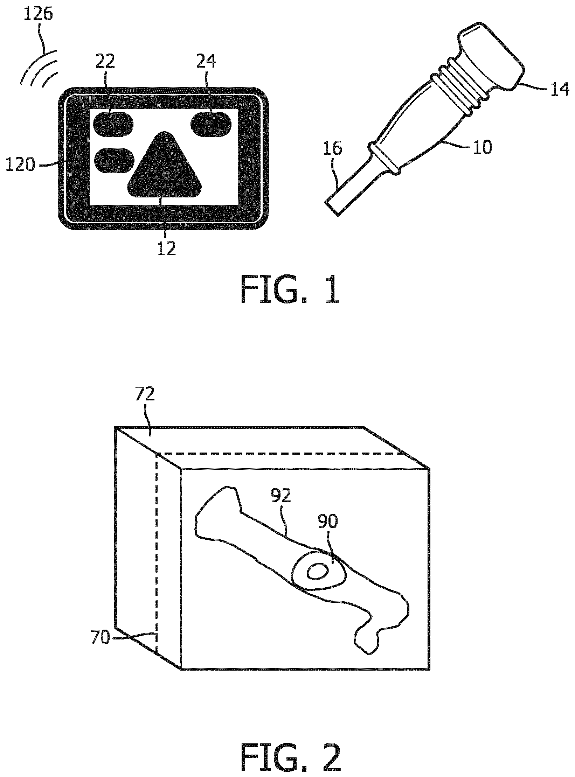

Referring to FIGURE 1, an ultrasound system of the present invention is shown. The system comprises a tablet ultrasound system with a touchscreen 120. A suitable commercial system with these characteristics is the Lumify^{™} ultrasound system, available from Philips Healthcare of Andover, MA. The touchscreen display will display an ultrasound image 12 as well as patient and system information 22 and touchscreen controls 24 by which a user controls the operation of the ultrasound system. To the right of the tablet system is an ultrasound probe 10 which transmits and receives ultrasonic energy with a two-dimensional transducer array located at its distal end 14. The two-dimensional array is capable of electronically scanning and acquiring echo signals for imaging over a volumetric region of a subject. Three-dimensional imaging may also be performed with a transducer having an oscillating one-dimensional array transducer. The ultrasound probe 10 is coupled either by a cable or wirelessly to the tablet ultrasound system, which is capable of Bluetooth and Wifi communication as indicated by waves 126. The probe 10 is shown with a stub antenna 16 at its proximal end for communication with the tablet ultrasound system.

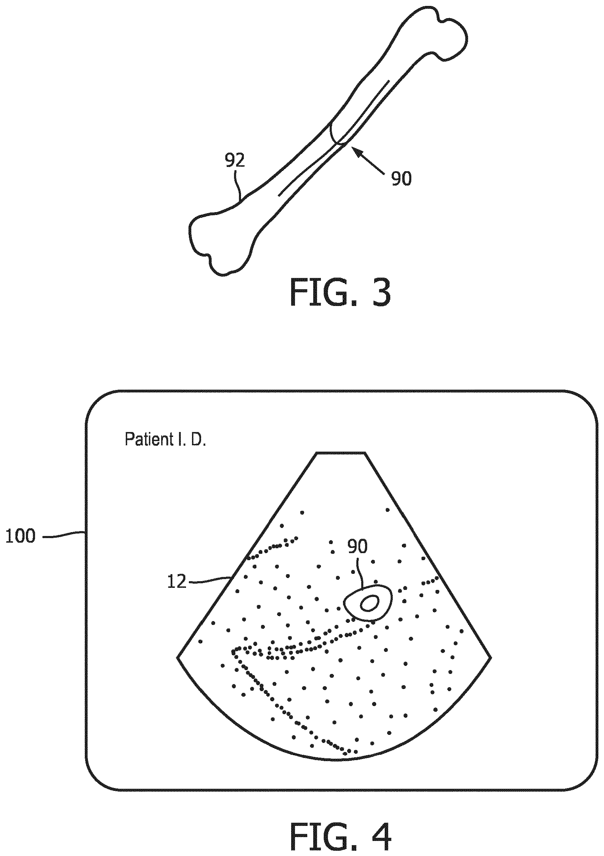

FIGURE 2 depicts a volume 72 of spatially arranged image voxels produced from echoes acquired by the ultrasound probe 10 for 3D imaging. In this example the volume of image data contains image data of a fetal femur 92 which was in the scanning region of the probe. The femur is shown in dotted phantom, depicting that the bone is obscured inside the volume by surrounding voxels of tissue. The longitudinal dimension of the femur extends from the front to the back of the volume 72. A cut plane 70 through the volume of image data is also illustrated. In this example the plane 70 of pixels of image data intersects the femur 90. Thus, the image produced from the pixels in image plane 70 will include a cross-sectional area 90 of the femur bone 92. FIGURE 3 is a picture of a femur bone 92 in perspective which indicates the location 90 of the slice through the bone which is in image plane 70 in FIGURE 2.

With this as background, FIGURE 4 shows an ultrasound system display 100 displaying a planar ultrasound image 12. This image typifies how an image of slice plane 70 will appear, including the cross-sectional view 90 of the femur 92 surrounded by pixels of the tissue located around the bone. In an implementation of the present invention, a user touches the section 90 of the femur on the display which is visible in the image. Touching the touchscreen sends a signal to the ultrasound system, indicating to the system a location in the image data around which the system is to analyze the volumetric image data to find an image plane containing a longitudinal view of the femur. The system already knows that the user is conducting an obstetrical exam, which was made known to the system when the user selected an obstetrical probe 10. Optionally, the user may also indicate to the system that it is a fetal femur bone which is to be located. An onboard image extraction processor now explores the volumetric image data in differently oriented planes which intersect the location in the image data marked by the touch of the user on bone section 90 on the touchscreen display until a plane containing a longitudinal image of the femur bone is found.

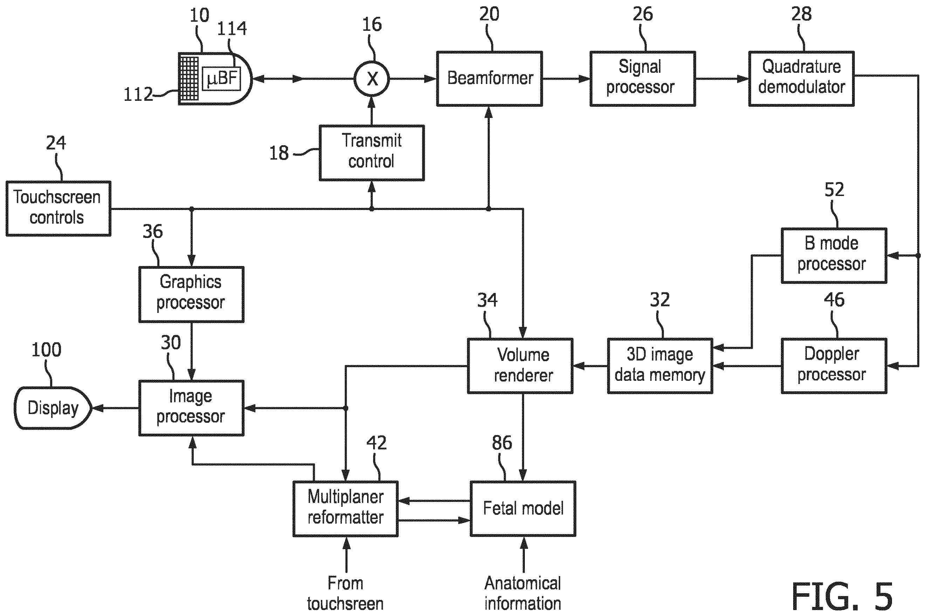

One implementation of an ultrasound system configured to perform this analysis and produce the desired image is shown in block diagram form in FIGURE 5. The ultrasound system of FIGURE 5 is provided for illustration purposes and is not within the scope of the claims. A transducer array 112 is provided in an ultrasound probe 10 for transmitting ultrasonic waves and receiving echo information over a volumetric region of the body. The transducer array 112 may be a two-dimensional array of transducer elements capable of electronically scanning in two or three dimensions, in both elevation (in 3D) and azimuth, as shown in the drawing. Alternatively, the transducer may be a one-dimensional array capable of scanning image planes which is oscillated back and forth to sweep the image plane through a volumetric region and thereby scan the region for three-dimensional imaging, such as that described in US Pat. 7,497,830 (Li et al.) A two-dimensional transducer array 112 is coupled to a microbeamformer 114 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled by the probe cable to a transmit/receive (T/R) switch 16 which switches between transmission and reception and protects the main system beamformer 20 from high energy transmit signals. The transmission of ultrasonic beams from the transducer array 112 under control of the microbeamformer 114 is directed by a transmit controller 18 coupled to the T/R switch and the beamformer 20, which receives input from the user's operation of the user interface or controls 24 on the touchscreen display. Among the transmit characteristics controlled by the transmit controller are the spacing, amplitude, phase, and polarity of transmit waveforms. Beams formed in the direction of pulse transmission may be steered straight ahead from the transducer array, or at different angles for a wider sector field of view, the latter being typical of most obstetrical imaging probes.

The echoes received by a contiguous group of transducer elements are beamformed by appropriately delaying them and then combining them. The partially beamformed signals produced by the microbeamformer 114 from each patch are coupled to a main beamformer 20 where partially beamformed signals from individual patches of transducer elements are delayed and combined into a fully beamformed coherent echo signal. For example, the main beamformer 20 may have 128 channels, each of which receives a partially beamformed signal from a patch of 12 transducer elements. In this way the signals received by over 1500 transducer elements of a two-dimensional array transducer can contribute efficiently to a single beamformed signal.

The coherent echo signals undergo signal processing by a signal processor 26, which includes filtering by a digital filter and noise reduction as by spatial or frequency compounding. The digital filter of the signal processor 26 can be a filter of the type disclosed in U.S. Patent No. 5,833,613 (Averkiou et al.), for example. The processed echo signals are demodulated into quadrature (I and Q) components by a quadrature demodulator 28, which provides signal phase information and can also shift the signal information to a baseband range of frequencies.

The beamformed and processed coherent echo signals are coupled to a B mode processor 52 which produces a B mode image of structure in the body such as tissue. The B mode processor performs amplitude (envelope) detection of quadrature demodulated I and Q signal components by calculating the echo signal amplitude in the form of (I²+Q²)^{½}. The quadrature echo signal components are also coupled to a Doppler processor 46, which stores ensembles of echo signals from discrete points in an image field which are then used to estimate the Doppler shift at points in the image with a fast Fourier transform (FFT) processor. The Doppler shift is proportional to motion at points in the image field, e.g., blood flow and tissue motion. For a color Doppler image, which may be formed for analysis of fetal blood flow, the estimated Doppler flow values at each point in a blood vessel are wall filtered and converted to color values using a look-up table. Either the B mode image or the Doppler image may be displayed alone, or the two shown together in anatomical registration in which the color Doppler overlay shows the blood flow in tissue and vessels in the imaged region.

The B mode image signals and the Doppler flow values when used are coupled to a 3D image data memory, which stores the image data in x, y, and z addressable memory locations corresponding to spatial locations in a scanned volumetric region of a subject. This volumetric image data is coupled to a volume renderer 34 which converts the echo signals of a 3D data set into a projected 3D image as viewed from a given reference point as described in US Pat. 6,530,885 (Entrekin et al.) The reference point, the perspective from which the imaged volume is viewed, may be changed by a control on the touchscreen display, which enables the volume to be tilted or rotated to diagnose the region from different viewpoints.

The volumetric image data used to produce the volume rendering is coupled to an image extraction processor which in this implementation is a fetal model 86 (which is not within the scope of the claims). The fetal model is a processor and memory which stores a library of differently sized and/or shaped models in data form of typical structures of interest in a fetal exam. The library may contain different sets of models, each representing typical fetal structure at a particular age of fetal development, such as the first and second trimesters of development, for instance. The models are data representing meshes of bones of the fetal skeleton and skin (surface) of a developing fetus. The meshes of the bones are interconnected as are the actual bones of a skeleton so that their relative movements and ranges of articulation are constrained in the same manner as are those of an actual skeletal structure. Similarly the surface mesh is constrained to be within a certain range of distance of the bones it surrounds. When the user has informed the system of known anatomical information, such as the bone to be identified is believed to be a femur of a fetus in the second trimester, this information is coupled to the fetal model and used to select a particular model from the library as the starting point for analysis. The models are deformable within constraint limits, *e.g.*, fetal age, by altering parameters of a model to warp the model, such as an adaptive mesh representing an approximate surface of a typical femur, and thereby fit the model by deformation to structural landmarks in the volumetric image data set. An adaptive mesh model is desirable because it can be warped within the limits of its mesh continuity and other constraints in an effort to fit the deformed model to structure in different image planes intersecting the identified bone location 90. This process is continued by an automated shape processor until data is found in a plane which can be fitted by the model and thus identified as the desired anatomy. The planes in the volumetric image data which are examined may be selected by the fetal model operating on the volumetric image data provided by the volume renderer 34, when the bone model is configured to do this. Alternatively, a series of differently oriented image planes intersecting the specified location 90 can be extracted from the volume data by a multiplanar reformatter 42 and provided to the fetal model 86 for analysis and fitting. The multiplanar reformatter selects echo data which are received from points in a common plane in a volumetric region of the body which can be displayed as an ultrasonic image of that plane, as described in US Pat. 6,443,896 (Detmer). In the instant system the multiplanar reformatter is programmed to couple to the fetal model a sequence of differently oriented planes of image data which intersect the location marked by the user's touch until a plane is found with image data fitted by the model. In either case, the identification of the plane of an image with the desired anatomy is coupled back to the multiplanar reformatter for display of the desired image plane. The image extraction processor may also provide and/or seek verification of the anatomy being identified, as by displaying a message "Femur?" to the user when it appears that the user is seeking to display the femur and verification of this is desired either by the processor or as reassurance to the user. The foregoing model deformation and fitting is explained in further detail in international patent application number WO 2015/019299 (Mollus et al.) entitled "MODEL-BASED SEGMENTATION OF AN ANATOMICAL STRUCTURE." See also international patent application number WO 2010/150156 (Peters et al.) entitled "ESTABLISHING A CONTOUR OF A STRUCTURE BASED ON IMAGE INFORMATION" and US pat. appl. pub. no. 2017/0128045 (Roundhill et al.) entitled "TRANSLATION OF ULTRASOUND ARRAY RESPONSIVE TO ANATOMICAL ORIENTATION."

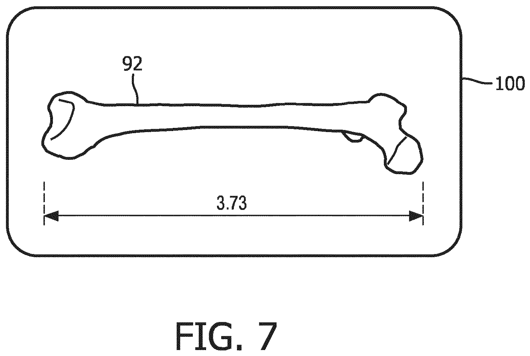

Once the orientation coordinates of an image plane containing the desired anatomy has been found, in this example a longitudinal view of a femur bone, this information is coupled to the multiplanar reformatter by the fetal bone model which selects that plane of data from the volumetric image data for display. The planar image data is coupled to an image processor 30 for scan conversion if necessary and further enhancement, buffering and temporary storage for display on an image display 40. In a preferred implementation the image processor also adds the desired measurement to the image, which is readily done as ultrasonic image data is spatially accurate. A graphics processor 36 produces a display overlay containing measurement graphics and the measurement together with the image of the desired fetal bone 92 as shown on image display 100 in FIGURE 7. If desired, the ultrasound system can automatically label the identified structure as a femur, and can also be configured to automatically call up the fetal age estimation program and enter the bone measurement into the program for expedited fetal age estimation.

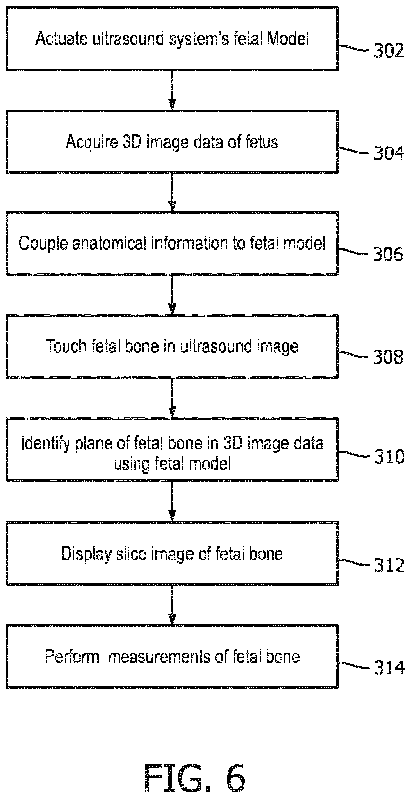

A method for operating the ultrasound system of FIGURE 5 to extract an image of desired fetal anatomy from volumetric image data is illustrated in FIGURE 6. In step 302 the fetal model of the ultrasound system is actuated. In step 304 3D (volumetric) image data of a fetus is acquired. In step 306 known anatomical information is optionally coupled to the fetal model, such as the particular bone to be identified and the age (trimester) of the fetus. In step 308 a user touches the desired fetal bone on a touchscreen display of an ultrasound image in which at least a portion of the bone is visible. This touch identification is used by a fetal model in step 310 to identify an image plane containing the desired bone in the 3D image data. In step 312 a slice image of an identified image plane of the desired fetal bone in longitudinal view is displayed. In step 314 measurements of the fetal bone are performed which, optionally, may be done automatically as described above.

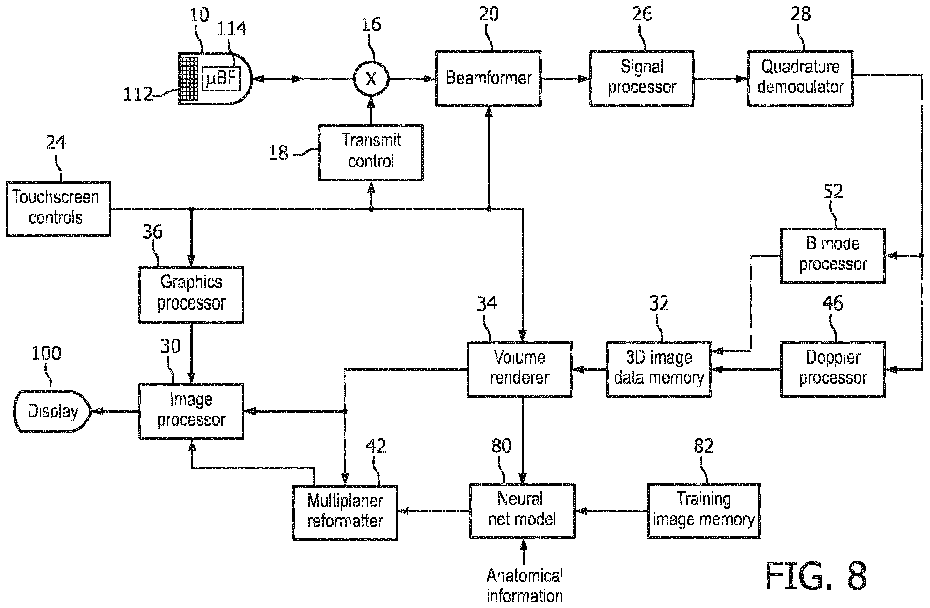

FIGURE 8 illustrates in block diagram form an ultrasound system comprising a second implementation of the present invention. In the system of FIGURE 8, system elements which were shown and described in FIGURE 5 are used for like functions and operations and will not be described again. In the system of FIGURE 8 the image extraction processor comprises a neural net model 80. A neural net model makes use of a development in artificial intelligence known as "deep learning." Deep learning is a rapidly developing branch of machine learning algorithms that mimic the functioning of the human brain in analyzing problems. The human brain recalls what was learned from solving a similar problem in the past, and applied that knowledge to solve a new problem. Exploration is underway to ascertain possible uses of this technology in a number of areas such as pattern recognition, natural language processing and computer vision. Deep learning algorithms have a distinct advantage over traditional forms of computer programming algorithms in that they can be generalized and trained to recognize image features by analyzing image samples rather than writing custom computer code. The anatomy visualized in an ultrasound system would not seem to readily lend itself to automated image recognition, however. Every person is different, and anatomical shapes, sizes, positions and functionality vary from person to person. Furthermore, the quality and clarity of ultrasound images will vary even when using the same ultrasound system. That is because body habitus will affect the ultrasound signals returned from the interior of the body which are used to form the images. Scanning a fetus through the abdomen of an expectant mother will often result in greatly attenuated ultrasound signals and poorly defined anatomy in the fetal images. Nevertheless, the system described in this application has demonstrated the ability to use deep learning technology to recognize anatomy in fetal ultrasound images through processing by a neural network model. The neural network model is first trained by presenting to it a plurality of images of known anatomy, such as fetal images with known fetal structure which is identified to the model. Once trained, live images acquired by a user during a fetal exam are analyzed by the neural net model in real time, which identifies the anatomy in the images.

Deep learning neural net models comprise software which may be written by a software designer, and are also publicly available from a number of sources. In the ultrasound system of FIGURE 8, the neural net model software is stored in a digital memory. An application which can be used to build a neural net model called "NVidia Digits" is available at https://developer.nvidia.com/digits. NVidia Digits is a high-level user interface around a deep learning framework called "Caffe" which has been developed by the Berkley Vision and Learning Center, http://caffe.berkeleyvision.org/. A list of common deep learning frameworks suitable for use in an implementation of the present invention is found at https://developer.nvidia.com/deep-learning-frameworks. Coupled to the neural net model 80 is a training image memory 82, in which ultrasound images of known fetal anatomy including fetal bones are stored and used to train the neural net model to identify that anatomy in 3D (volumetric) ultrasound image data sets. Once the neural net model is trained by a large number of known fetal images, the neural net model receives a volume image data set of a fetus from the volume renderer 34. The neural net model may receive other cues in the form of anatomical information such as the fact that an obstetrical exam is being performed and the trimester of the fetus, as described above. The neural net model also receives the locational signal generated by a user touching a portion of the desired anatomy on a touchscreen display, a femur bone in this example. The neural net model then analyzes regions including the identified location until a femur bone is identified in the volume image data. The coordinates of a plane containing longitudinal image data of the femur are coupled to the multiplanar reformatter 42, which extracts the desired femur image from the volumetric image data set and forwards it to the image processor for display as shown in FIGURE 7. As before, the ultrasound system may be conditioned to automatically label and/or measure the bone, display the measurement, and couple the measurement information to another program such as a gestational age estimation program.

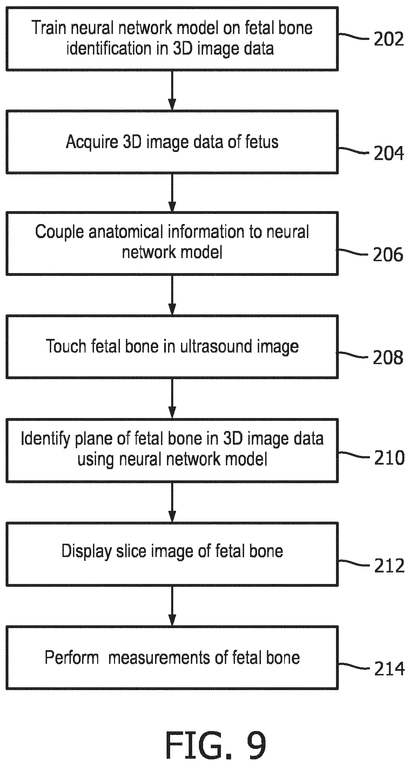

A method for operating the ultrasound system of FIGURE 8 to extract an image of desired fetal anatomy from volumetric image data is illustrated in FIGURE 9. In step 202 a neural network model is trained to identify fetal bones in 3D fetal image data. In step 204 3D (volumetric) image data of a fetus is acquired. In step 206 known anatomical information is optionally coupled to the fetal bone model, such as the particular bone to be identified and the age (trimester) of the fetus. In step 208 a user touches the desired fetal bone on a touchscreen display of an ultrasound image in which at least a portion of the bone is visible. This touch identification of location in an image is used by a neural network model in step 210 to identify an image plane containing the desired bone in the 3D image data. In step 212 a slice image of an identified image plane of the desired fetal bone is displayed. In step 214 measurements of the fetal bone are performed which, optionally, may be done automatically as described above.

Variations of the systems and methods described above will readily occur to those skilled in the art. A number of system components shown in FIGURES 5 and 8 can be located in the probe case. Some Lumify probes, for example, contain components from the transducer through the B mode processor, outputting to the tablet display detected image signals over a USB cable. This methodology can be extended to include even additional components in the probe, if desired, such as the 3D image data memory and volume rendering software. Thus, a number of components which are described above as "system" components may alternatively be located in the ultrasound probe.

The techniques of the present invention can be used in other diagnostic areas besides obstetrics. For instance, numerous ultrasound exams require standard views of anatomy for diagnosis. In diagnoses of the kidney, a standard view is a coronal image plane of the kidney. In cardiology, twochamber, three-chamber, and four-chamber views of the heart are standard views. A neural network model can be trained to recognize such views in 3D image data sets of the heart and then be used to select image planes of desired views from volumetric data and display them to a clinician. Other applications will readily occur to those skilled in the art.

It should be noted that an ultrasound system suitable for use in an implementation of the present invention, and in particular the component structure of the ultrasound systems of FIGURES 5 and 8, may be implemented in hardware, software or a combination thereof. The various embodiments and/or components of an ultrasound system, for example, the fetal bone model and deep learning software modules, or components, processors, and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system or the data network for importing training images. The computer or processor may also include a memory. The memory devices such as the 3D image data memory 32, the training image memory, and the memory storing fetal bone model libraries may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules such as a neural network model module, a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasonic diagnostic imaging system for extracting a desired view of a fetal anatomy from volume image data which includes the fetal anatomy comprising:
an ultrasound probe (10) adapted to acquire volume image data of a fetus which includes image data of a desired anatomy (92);
a B mode processor (52) adapted to produce B mode image data from the volume image data;
a display (100) adapted to display an ultrasound image (12) from the acquired image data showing at least a portion of the desired anatomy on a touchscreen display;
an image extraction processor comprising a neural network model (80) trained with known images of the desired anatomy, wherein the neural network model (80) is adapted to:
- receive the volume image data and a locational signal generated by a user touch of the desired anatomy on the touchscreen display,
- responsive to the received volume image data and user touch, analyze regions of the volume image data including an identified anatomical location indicated by the user touch until the desired anatomy is recognized in the B mode image data of the volume image data,
- recognize an image plane containing the desired anatomy recognized in the B mode image data of the volume image data, and
- extract said recognized image plane of the desired anatomy from the volume image data;
wherein the display is further adapted to display the extracted image plane of the desired anatomy.

2. The ultrasonic diagnostic imaging system of Claim 1, further comprising a multiplanar reformatter (42), responsive to the volume image data and the recognition of the image plane containing the desired anatomy by the neural network model (80), which is adapted to produce an image plane of image data containing the desired anatomy from the volume image data.

3. The ultrasonic diagnostic imaging system of Claim 1 or 2, wherein the system is further responsive to an extraction of an image of the desired anatomy by the neural network model and adapted to produce a measurement of the desired anatomy.

4. The ultrasonic diagnostic imaging system of any of Claims 1 to 3, wherein the desired anatomy (92) is a fetal bone;
wherein the image (12) of the desired anatomy is an image of the fetal bone; and
wherein the display is further adapted to display a measurement of the fetal bone.

5. The ultrasonic diagnostic imaging system of Claim 4, wherein the system is further adapted to use the measurement of the fetal bone in a fetal age estimation.

6. The ultrasonic diagnostic imaging system of any of Claims 3 to 5, wherein the extracted image further comprises a standard view of the desired anatomy.

7. A method of producing a desired view of a fetal anatomy during ultrasound imaging comprising:
acquiring (204, 304) volume image data of a fetus which includes image data of the desired anatomy (92); producing Bmode image data from the volume image data using a Bmode processor;
displaying (212, 312) an image from the volume image data which includes at least a portion of the desired anatomy on a touchscreen display;
sensing (208, 308) a user touch on the at least a portion of the desired anatomy on the touchscreen display, and generating a locational signal responsive thereto;
extracting an image plane of the desired anatomy using an image extraction processor comprising a neural network model (80) trained with known images of the desired anatomy, wherein the extracting comprises:
- receiving the volume image data and the locational signal generated by the user touch of the desired anatomy on the touchscreen display,
- in response to the received volume image data and the user touch, analyzing, using the neural network model, regions of the volume image data including an identified anatomical location indicated by the user touch until the desired anatomy is recognized in B mode image data of the volume image data,
- recognizing, using the neural network model, an image plane containing the desired anatomy recognized in the B mode image data of the volume image data, and
- extracting, using the neural network model, the recognized image plane of the desired anatomy from the volume image data; and
displaying (212, 312) the extracted image plane of the desired anatomy.

8. The method of Claim 7, further comprising:
responsive to the volume image data and the recognition of the image plane containing the desired anatomy by the neural network model (80), using a multiplanar reformatter (42) to produce an image plane of image data containing the desired anatomy from the volume image data.

9. The method of Claim 7 or 8, further comprising:
responsive to an extraction of an image o: the desired anatomy by the neural network model (80), producing a measurement of the desired anatomy.

10. The method of any of claim 9, wherein
the desired anatomy (92) is a fetal bone;
wherein the image (12) of the desired anatomy is an image of the fetal bone; and
wherein the method further comprises displaying a measurement of the fetal bone on the display.

11. The method of Claim 10, further comprising:
using the measurement of the fetal bone in a fetal age estimation.

12. The method of any of Claims 9 to 11,
wherein the extracted image further comprises a standard view of the desired anatomy.

13. A tangible, non-transitory computer readable medium comprising instructions that, when executed by a processor included in the ultrasonic diagnostic imaging system of any of Claims 1-7, cause the processor to perform the method of any of Claims 7-12.

## Patentansprüche

1. Ultraschall-Diagnosebildgebungssystem zum Extrahieren einer gewünschten Ansicht einer fetalen Anatomie aus Volumenbilddaten, die die fetale Anatomie beinhalten, umfassend:
eine Ultraschallsonde (10), die dazu ausgelegt ist, Volumenbilddaten eines Fötus aufzunehmen, die Bilddaten einer gewünschten Anatomie (92) beinhalten;
einen B-Mode-Prozessor (52), der dazu ausgelegt ist, aus den Volumenbilddaten B-Mode-Bilddaten zu erzeugen;
eine Anzeige (100), die dazu ausgelegt ist, ein Ultraschallbild (12) aus den aufgenommenen Bilddaten anzuzeigen, das mindestens einen Teil der gewünschten Anatomie auf einer Berührungsbildschirmanzeige zeigt;
einen Bildextraktionsprozessor, der ein neuronales Netzwerkmodell (80) umfasst, das mit bekannten Bildern der gewünschten Anatomie trainiert wurde, wobei das neuronale Netzwerkmodell (80) ausgelegt ist für:
- Empfangen der Volumenbilddaten und eines Positionssignals, das durch eine Berührung der gewünschten Anatomie auf der Berührungsbildschirmanzeige durch den Benutzer generiert wird,
- als Reaktion auf die empfangenen Volumenbilddaten und die Berührung des Benutzers, Analysieren von Bereichen der Volumenbilddaten, die eine durch die Berührung des Benutzers identifizierte anatomische Position beinhalten, bis die gewünschte Anatomie in den B-Mode-Bilddaten der Volumenbilddaten erkannt wird,
- Erkennen einer Bildebene, die die gewünschte Anatomie enthält, die in den B-Mode-Bilddaten der Volumenbilddaten erkannt wurde, und
- Extrahieren der erkannten Bildebene der gewünschten Anatomie aus den Volumenbilddaten;
wobei die Anzeige ferner dazu ausgelegt ist, die extrahierte Bildebene der gewünschten Anatomie anzuzeigen.

2. Ultraschall-Diagnosebildgebungssystem nach Anspruch 1, das ferner einen multiplanaren Reformatter (42) als Reaktion auf die Volumenbilddaten und die Erkennung der Bildebene, die die gewünschte Anatomie enthält, durch das neuronale Netzwerkmodell (80) umfasst, der dazu ausgelegt ist, aus den Volumenbilddaten eine Bildebene von Bilddaten zu erzeugen, die die gewünschte Anatomie enthalten.

3. Ultraschall-Diagnosebildgebungssystem nach Anspruch 1 oder 2, wobei das System ferner auf eine Extraktion eines Bilds der gewünschten Anatomie durch das neuronale Netzwerkmodell reagiert und dazu ausgelegt ist, eine Messung der gewünschten Anatomie zu erzeugen.

4. Ultraschall-Diagnosebildgebungssystem nach einem der Ansprüche 1 bis 3, wobei es sich bei der gewünschten Anatomie (92) um einen fetalen Knochen handelt;
wobei es sich bei dem Bild (12) der gewünschten Anatomie um ein Bild des fetalen Knochens handelt; und
wobei die Anzeige ferner dazu ausgelegt ist, eine Messung des fetalen Knochens anzuzeigen.

5. Ultraschall-Diagnosebildgebungssystem nach Anspruch 4, wobei das System ferner dazu ausgelegt ist, die Messung des fetalen Knochens bei einer Einschätzung des fetalen Alters zu nutzen.

6. Ultraschall-Diagnosebildgebungssystem nach einem der Ansprüche 3 bis 5, wobei das extrahierte Bild ferner eine Standardansicht der gewünschten Anatomie umfasst.

7. Verfahren zum Erzeugen einer gewünschten Ansicht einer fetalen Anatomie während Ultraschallbildgebung, umfassend:
Aufnehmen (204, 304) von Volumenbilddaten eines Fötus, die Bilddaten der gewünschten Anatomie (92) beinhalten; Erzeugen von B-Mode-Bilddaten aus den Volumenbilddaten unter Verwendung eines B-Mode-Prozessors;
Anzeigen (212, 312) eines Bilds aus den Volumenbilddaten, das mindestens einen Teil der gewünschten Anatomie beinhaltet, auf einer Berührungsbildschirmanzeige;
Erfassen (208, 308) einer Berührung des Benutzers auf dem mindestens einen Teil der gewünschten Anatomie auf der Berührungsbildschirmanzeige und Generieren eines Positionssignals als Reaktion darauf;
Extrahieren einer Bildebene der gewünschten Anatomie unter Verwendung eines Bildextraktionsprozessors, der ein neuronales Netzwerkmodell (80) umfasst, das mit bekannten Bildern der gewünschten Anatomie trainiert wurde, wobei das Extrahieren umfasst:
- Empfangen der Volumenbilddaten und des Positionssignals, das durch die Berührung der gewünschten Anatomie auf der Berührungsbildschirmanzeige durch den Benutzer generiert wird,
- als Reaktion auf die empfangenen Volumenbilddaten und die Berührung des Benutzers, Analysieren, unter Verwendung des neuronalen Netzwerkmodells, von Bereichen der Volumenbilddaten, die eine durch die Berührung des Benutzers identifizierten anatomischen Position beinhalten, bis die gewünschte Anatomie in B-Mode-Bilddaten der Volumenbilddaten erkannt wird,
- Erkennen, unter Verwendung des neuronalen Netzwerkmodells, einer Bildebene, die die gewünschte Anatomie enthält, die in den B-Mode-Bilddaten der Volumenbilddaten erkannt wurde, und
- Extrahieren, unter Verwendung des neuronalen Netzwerkmodells, der erkannten Bildebene der gewünschten Anatomie aus den Volumenbilddaten; und
Anzeigen (212, 312) der extrahierten Bildebene der gewünschten Anatomie.

8. Verfahren nach Anspruch 7, das ferner umfasst:
als Reaktion auf die Volumenbilddaten und die Erkennung der Bildebene, die die gewünschte Anatomie enthält, durch das neuronale Netzwerkmodell (80), Verwenden eines multiplanaren Reformatters (42), um aus den Volumenbilddaten eine Bildebene von Bilddaten zu erzeugen, die die gewünschte Anatomie enthalten.

9. Verfahren nach Anspruch 7 oder 8, das ferner umfasst:
als Reaktion auf eine Extraktion eines Bilds der gewünschten Anatomie durch das neuronale Netzwerkmodell (80), Erzeugen einer Messung der gewünschten Anatomie.

10. Verfahren nach Anspruch 9, wobei es sich bei der gewünschten Anatomie (92) um einen fetalen Knochen handelt;
wobei es sich bei dem Bild (12) der gewünschten Anatomie um ein Bild des fetalen Knochens handelt; und
wobei das Verfahren ferner Anzeigen einer Messung des fetalen Knochens auf der Anzeige umfasst.

11. Verfahren nach Anspruch 10, das ferner umfasst:
Verwenden der Messung des fetalen Knochens bei einer Einschätzung des fetalen Alters.

12. Verfahren nach einem der Ansprüche 9 bis 11, wobei das extrahierte Bild ferner eine Standardansicht der gewünschten Anatomie umfasst.

13. Greifbares, nichtflüchtiges, computerlesbares Medium, das Anweisungen umfasst, die, wenn sie von einem in dem Ultraschall-Diagnosebildgebungssystem nach einem der Ansprüche 1-7 beinhalteten Prozessor ausgeführt werden, den Prozessor veranlassen, das Verfahren nach einem der Ansprüche 7-12 durchzuführen.

## Revendications

1. Système d'imagerie diagnostique ultrasonore permettant d'extraire une vue souhaitée de l'anatomie fœtale à partir de données d'images volumiques incluant l'anatomie fœtale comprenant :
une sonde à ultrasons (10) adaptée pour acquérir des données d'image volumique d'un fœtus qui inclut des données d'image d'une anatomie souhaitée (92) ;
un processeur de mode B (52) adapté pour produire des données d'image en mode B à partir des données d'image de volume ;
un écran (100) adapté pour afficher une image ultrasonore (12) à partir des données d'image acquises montrant au moins une partie de l'anatomie souhaitée sur un écran tactile ;
un processeur d'extraction d'images comprenant un modèle de réseau neuronal (80) entraîné avec des images connues de l'anatomie souhaitée, dans lequel le modèle de réseau neuronal (80) est adapté pour :
- recevoir les données d'image volumique et un signal de localisation généré par un toucher de l'utilisateur sur l'anatomie souhaitée sur l'écran tactile,
- en réponse aux données d'image volumique reçues et au toucher de l'utilisateur, analyser les régions des données d'image volumique, y compris un emplacement anatomique identifié indiqué par le toucher de l'utilisateur, jusqu'à ce que l'anatomie souhaitée soit reconnue dans les données d'image en mode B des données d'image volumique,
- reconnaître un plan d'image contenant l'anatomie souhaitée reconnue dans les données d'image en mode B des données d'image volumique, et
- extraire ledit plan d'image reconnu de l'anatomie souhaitée à partir des données d'image volumique ;
dans lequel l'écran est en outre adapté pour afficher le plan d'image extrait de l'anatomie souhaitée.

2. Système d'imagerie diagnostique ultrasonique selon la revendication 1, comprenant en outre un reformateur multiplan (42), sensible aux données d'image volumique et à la reconnaissance du plan d'image contenant l'anatomie souhaitée par le modèle de réseau neuronal (80), qui est adapté pour produire un plan d'image de données d'image contenant l'anatomie souhaitée à partir des données d'image volumique.

3. Système d'imagerie diagnostique ultrasonore selon la revendication 1 ou 2, dans lequel le système est en outre sensible à l'extraction d'une image de l'anatomie souhaitée par le modèle de réseau neuronal et adapté pour produire une mesure de l'anatomie souhaitée.

4. Système d'imagerie diagnostique ultrasonore selon l'une quelconque des revendications 1 à 3, dans lequel l'anatomie souhaitée (92) est un os fœtal ;
dans lequel l'image (12) de l'anatomie souhaitée est une image de l'os fœtal ; et
dans lequel l'écran est en outre adapté pour afficher une mesure de l'os fœtal.

5. Système d'imagerie diagnostique ultrasonore selon la revendication 4, dans lequel le système est en outre adapté pour utiliser la mesure de l'os fœtal dans une estimation de l'âge fœtal.

6. Système d'imagerie diagnostique ultrasonore selon l'une quelconque des revendications 3 à 5, dans lequel l'image extraite comprend en outre une vue standard de l'anatomie souhaitée.

7. Procédé permettant de produire une vue souhaitée de l'anatomie fœtale lors d'une imagerie ultrasonore, comprenant :
l'acquisition (204, 304) de données d'image volumique d'un fœtus qui incluent des données d'image de l'anatomie souhaitée (92) ; la production de données d'image Bmode à partir des données d'image volumique à l'aide d'un processeur Bmode ;
l'affichage (212, 312) d'une image à partir des données d'image volumique qui inclut au moins une partie de l'anatomie souhaitée sur un écran tactile ;
la détection (208, 308) d'un contact de l'utilisateur sur l'au moins une partie de l'anatomie souhaitée sur l'écran tactile, et la génération d'un signal de localisation en réponse à celui-ci ;
l'extraction d'un plan d'image de l'anatomie souhaitée à l'aide d'un processeur d'extraction d'images comprenant un modèle de réseau neuronal (80) entraîné avec des images connues de l'anatomie souhaitée, dans lequel l'extraction comprend :
- la réception des données d'image volumique et du signal de localisation généré par le toucher de l'utilisateur sur l'anatomie souhaitée sur l'écran tactile,
- en réponse aux données d'image volumique reçues et au toucher de l'utilisateur, l'analyse, à l'aide du modèle de réseau neuronal, des régions des données d'image volumique incluant une localisation anatomique identifiée indiquée par le toucher de l'utilisateur jusqu'à ce que l'anatomie souhaitée soit reconnue dans les données d'image en mode B des données d'image volumique,
- la reconnaissance, à l'aide du modèle de réseau neuronal, d'un plan d'image contenant l'anatomie souhaitée reconnue dans les données d'image en mode B des données d'image volumique, et
- l'extraction, à l'aide du modèle de réseau neuronal, du plan d'image reconnu de l'anatomie souhaitée à partir des données d'image volumique ; et
l'affichage (212, 312) du plan d'image extrait de l'anatomie souhaitée.

8. Procédé selon la revendication 7, comprenant en outre :
en réponse aux données d'image volumique et à la reconnaissance du plan d'image contenant l'anatomie souhaitée par le modèle de réseau neuronal (80), l'utilisation d'un reformateur multiplan (42) pour produire un plan d'image de données d'image contenant l'anatomie souhaitée à partir des données d'image volumique.

9. Procédé selon la revendication 7 ou 8, comprenant en outre :
en réponse à l'extraction d'une image o: l'anatomie souhaitée par le modèle de réseau neuronal (80), la production d'une mesure de l'anatomie souhaitée.

10. Procédé selon l'une quelconque des revendications 9, dans lequel l'anatomie souhaitée (92) est un os fœtal ;
dans lequel l'image (12) de l'anatomie souhaitée est une image de l'os fœtal ; et
dans lequel le procédé comprend en outre l'affichage d'une mesure de l'os fœtal sur l'écran.

11. Procédé selon la revendication 10, comprenant en outre :
l'utilisation de la mesure de l'os fœtal pour estimer l'âge fœtal.

12. Procédé selon l'une quelconque des revendications 9 à 11, dans lequel l'image extraite comprend en outre une vue standard de l'anatomie souhaitée.

13. Support tangible, non transitoire et lisible par ordinateur comprenant des instructions qui, lorsqu'elles sont exécutées par un processeur inclus dans le système d'imagerie diagnostique ultrasonore selon l'une quelconque des revendications 1-7, amènent le processeur à exécuter le procédé selon l'une quelconque des revendications 7-12.
